# FASCICULE DE BREVET EUROPEEN

(11) **EP 3 352 693 B1**
(45) Date de publication et mention de la délivrance du brevet: **23.10.2019**
(21) Numéro de dépôt: 16775590.9
(22) Date de dépôt: 22.09.2016
(51) Int. Cl.: A61B 17/72

(54) **IMPLANT INTRAMÉDULLAIRE POUR LE BLOCAGE DE L'ARTICULATION ENTRE DEUX PHALANGES ET SON MATÉRIEL DE POSE**
INTRAMEDULLÄRES IMPLANTAT ZUR RUHIGSTELLUNG DES GELENKS ZWISCHEN ZWEI PHALANGEN UND AUSRÜSTUNG ZUR ANPASSUNG DES BESAGTEN IMPLANTATS IN SITU
INTRAMEDULLARY IMPLANT FOR IMMOBILIZING THE ARTICULATON BETWEEN TWO PHALANGES, AND EQUIPMENT FOR FITTING SAID IMPLANT IN PLACE

(30) Priorité: 23.09.2015 FR 1558961
(43) Date de publication de la demande: 01.08.2018
(73) Titulaire: Newclip International, 2163 Luxembourg (LU)
(72) Inventeur: BEAUDET, Philippe, 69530 Brignais (FR); PODGORSKI, Jean-Pierre, 49230 St-Crespin sur Moine (FR); LARCHE, Grégoire, 49300 Cholet (FR)
(74) Mandataire: Jacobacci Coralis Harle
(86) Numéro de dépôt international: PCT/EP2016/072596
(87) Numéro de publication internationale: WO 2017/050927

(56) Documents cités:
- WO-A1-2013/177252
- WO-A2-2015/073942
- US-A1- 2013 123 862

## Description

### DOMAINE TECHNIQUE AUQUEL SE RAPPORTE L'INVENTION

La présente invention concerne le domaine technique des dispositifs d'ostéosynthèse ou d'arthrodèse.

Elle concerne plus particulièrement un implant intramédullaire destiné aux arthrodèses interphalangiennes, pour le blocage de l'articulation entre deux phalanges de la main ou du pied.

Et elle concerne encore un dispositif d'ostéosynthèse ou d'arthrodèse comportant un tel implant intramédullaire et son matériel de pose.

### ARRIERE-PLAN TECHNOLOGIQUE

Certaines pathologies des doigts ou des orteils nécessitent la mise en oeuvre de dispositifs d'ostéosynthèse pour bloquer l'articulation entre deux phalanges.

C'est par exemple le cas pour la pathologie des orteils en griffes consistant en une déformation permanente des orteils dans le plan sagittal.

On connait différents types d'implants chirurgicaux intramédullaires utilisés pour réaligner les phalanges concernées ou pour fixer leur orientation.

Par exemple, les documents FR-2 846 545, ou encore FR-2 884 406 proposent des implants constitués d'un corps allongé dans lequel une partie intermédiaire relie de manière rigide deux parties d'ancrage d'extrémités, aptes chacune à venir s'ancrer dans le canal médullaire des deux phalanges en regard que l'on souhaite traiter.

Ces parties d'ancrage d'extrémités consistent en des structures méplates du type à mémoire de forme pour optimiser la fixation au sein de l'os de réception.

L'implant décrit dans le document FR-2 884 406 comporte une première partie d'ancrage d'extrémité formée de deux bras ou ailettes et une seconde partie d'ancrage d'extrémité formée d'une boucle de matière, qui s'étendent toutes deux dans un même plan.

Les parties d'ancrage de l'implant du document FR-2 846 545 consistent toutes deux en des paires de bras ou tiges parallèles qui peuvent s'étendre dans le même plan, ou dans des plans inclinés l'un par rapport à l'autre, selon l'angle que l'on souhaite imposer aux phalanges.

Les documents WO-2015/073942 et US-2013/0123862 décrivent d'autres structures d'implants intramédullaires pour le blocage de l'articulation entre deux phalanges de la main ou du pied, constitués d'un corps allongé monobloc comprenant deux parties d'ancrage d'extrémités de forme générale plane, qui s'étendent dans des plans perpendiculaires entre eux.

Mais, à plus ou moins long terme, il arrive que ces dispositifs ne tiennent pas correctement dans l'os de réception, et il est alors nécessaire de mettre en oeuvre une nouvelle intervention chirurgicale pour changer l'implant.

De plus, les implants actuels sont fragiles et lorsqu'ils sont du type à mémoire de forme, leur expansion n'est pas toujours facile à contrôler.

### OBJET DE L'INVENTION

La présente invention a pour but de remédier à ces inconvénients en proposant un implant intramédullaire qui, une fois en place, dispose d'une bonne tenue dans le temps, tout en étant à la fois résistant et peu invasif.

Cet implant intramédullaire conforme à l'invention pour le blocage de l'articulation entre deux phalanges de la main ou du pied est constitué d'un corps allongé monobloc d'axe longitudinal **L** comprenant :
- une première partie d'ancrage d'extrémité, de forme générale plane, s'étendant dans un plan P1, apte à venir s'ancrer dans le canal médullaire de l'une desdites phalanges,
- une seconde partie d'ancrage d'extrémité, de forme générale plane, s'étendant dans un plan P2, apte à venir s'ancrer dans le canal médullaire de l'autre desdites phalanges, et
- une partie intermédiaire, reliant de manière rigide lesdites première et seconde parties d'ancrage d'extrémités,
lesquelles première et seconde parties d'ancrage d'extrémités s'étendent dans des plans P1 et P2 qui sont perpendiculaires ou sensiblement perpendiculaires entre eux,
laquelle première partie d'ancrage d'extrémité est en forme générale de U comportant une embase raccordée à ladite partie intermédiaire, et deux bras s'étendant parallèlement ou sensiblement parallèlement entre eux à partir de ladite embase,
et laquelle seconde partie d'ancrage d'extrémité est en forme de platine raccordée à ladite partie intermédiaire par une embase, laquelle platine est délimitée par deux faces parallèles entre elles et parallèles audit plan P2, par une extrémité libre et par deux bordures latérales, lesquelles bordures latérales comportent une pluralité de dents juxtaposées.

Dans ce cadre, au moins une réservation constituant un amincissement de matière est de préférence prévue au niveau de la zone de raccordement entre ladite embase et chacun desdits bras, pour optimiser l'ancrage au sein de l'os.

Selon une autre particularité, lesdits bras de la première partie d'ancrage d'extrémité sont chacun délimités par deux faces planes parallèles entre elles et parallèles au plan P1, par une bordure extérieure et par une bordure intérieure, lesdites bordures intérieures des deux bras étant en regard l'une de l'autre, et lesdites bordures extérieures des deux bras définissant les bordures latérales de ladite première partie d'ancrage d'extrémité.

Selon encore une autre caractéristique, lesdits bras disposent chacun d'une possibilité de mobilité élastique par rapport à ladite embase, cela dans ledit plan P1, et lesdits bras comportent chacun une bordure extérieure et une bordure intérieure, lesdites bordures intérieures des deux bras étant en regard l'une de l'autre et lesdites bordures extérieures des deux bras définissant les bordures latérales de ladite première partie d'ancrage d'extrémité ; en outre, lesdits bras ont chacun une forme générale cintrée dont la partie concave correspond à ladite bordure extérieure.

Toujours selon une caractéristique préférentielle, les extrémités libres des deux bras comportent chacune une pointe monobloc s'étendant en saillie au niveau de ladite bordure extérieure, lesdites pointes des deux bras s'étendant dans ledit plan P1 et dans des directions opposées.

De préférence, la platine de ladite seconde partie d'ancrage d'extrémité est de forme générale rectangulaire, et son extrémité libre est avantageusement en forme de pointe.

Selon une forme de réalisation préférée, l'implant intramédullaire comporte des moyens pour sa préhension par un matériel adapté, lesquels moyens consistent en un orifice traversant ménagé dans ladite platine, à proximité de ladite embase, et en deux appuis latéraux ménagés sur les côtés de ladite embase, entre ledit orifice traversant et ladite partie intermédiaire.

De son côté, la partie intermédiaire de l'implant comporte une structure monobloc qui s'étend dans un plan P3 perpendiculaire audit axe longitudinal L.

De préférence, cette structure monobloc est en forme générale de plateau centré sur ledit axe longitudinal L, et ce plateau est de préférence rectangulaire, comportant quatre réservations au niveau de ses angles.

Selon une autre caractéristique avantageuse, la première partie d'ancrage est adaptée pour constituer la partie proximale d'ancrage destinée à venir s'ancrer dans le canal médullaire de la phalange proximale de l'articulation ; de son côté, la seconde partie d'ancrage est adaptée pour constituer la partie distale d'ancrage, destinée à venir s'ancrer dans le canal médullaire de la phalange distale.

L'invention concerne encore un dispositif d'ostéosynthèse ou d'arthrodèse comportant :
a/ un implant intramédullaire selon la forme de réalisation préférée ci-dessus, et
b/ un dispositif de pose pour ledit implant intramédullaire, qui se présente sous la forme d'une pince de préhension munie de deux mâchoires articulées :
   - une première desdites mâchoires comportant une tige apte à venir se loger dans l'orifice traversant de la platine de ladite seconde partie d'ancrage d'extrémité, et
   - une seconde desdites mâchoires comportant deux bras parallèles ou sensiblement parallèles, aptes chacun à venir coopérer avec l'un desdits appuis latéraux de ladite seconde partie d'ancrage d'extrémité.

Ce dispositif d'ostéosynthèse ou d'arthrodèse comporte encore avantageusement, au moins :
- une fraise de résection comportant une partie active montée à l'extrémité d'une tige munie d'un manche,
- une râpe proximale comportant une partie active adaptée pour la préparation d'un logement intramédullaire dans la phalange proximale, en vue de la réception de la partie proximale d'ancrage de l'implant intramédullaire, et
- une râpe distale comportant une partie active adaptée pour la préparation d'un logement intramédullaire dans la phalange distale, en vue de la réception de la partie distale d'ancrage de l'implant intramédullaire.

### DESCRIPTION DETAILLEE D'UN EXEMPLE DE REALISATION

L'invention sera encore illustrée, sans être aucunement limitée, par la description suivante en association avec les dessins annexés dans lesquels :
- la figure 1 est une vue en perspective d'un implant intramédullaire conforme à l'invention ;
- la figure 2 est une vue de dessus de l'implant intramédullaire illustré sur la figure 1 ;
- la figure 3 est une vue de côté de l'implant intramédullaire illustré sur les figures 1 et 2 ;
- la figure 4 est une vue de côté d'une pince de préhension selon l'invention pour la pose de l'implant illustré sur les figures 1 à 3 ;
- les figures 5 à 7 sont des vues de côté illustrant les outils ou ancillaires utilisés pour la préparation du matériau osseux, lors de la pose de l'implant illustré sur les figures 1 à 3 ;
- la figure 8 est une vue de côté montrant la préhension de l'implant des figures 1 à 3 par la pince de préhension illustrée figure 4 ;
- la figure 9 est une vue agrandie illustrant le positionnement des tige et bras de la pince de préhension pour la préhension de l'implant intramédullaire des figures 1 à 3 ;
- les figures 10 et 11 sont des vues de côté illustrant, en deux étapes, la pose de l'implant intramédullaire au niveau de l'articulation des deux phalanges à bloquer ;
- la figure 12 est une vue de côté montrant l'implant intramédullaire en position entre les deux phalanges.

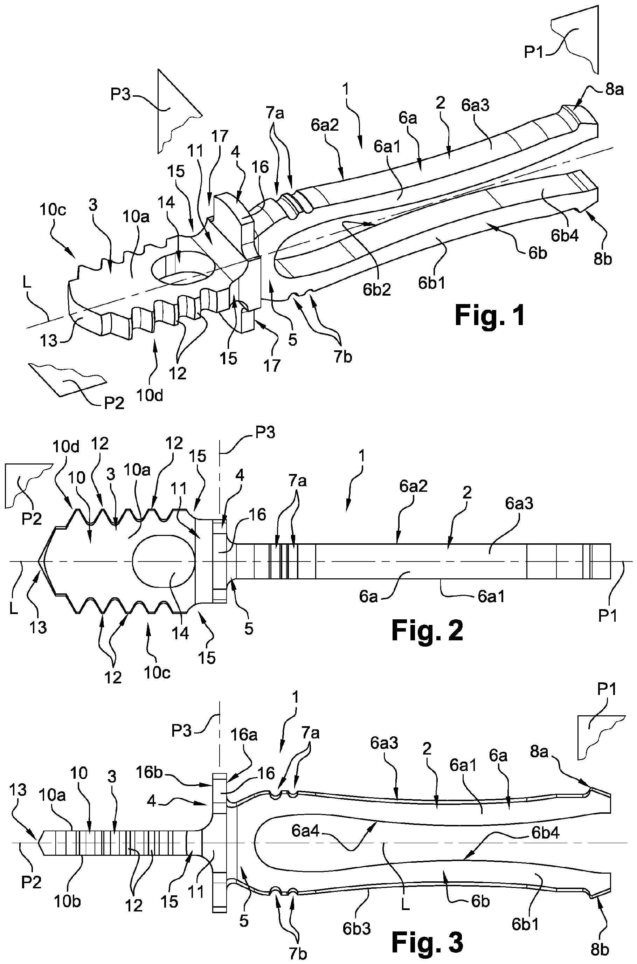

L'implant intramédullaire 1 illustré sur les figures 1 à 3 est adapté aux arthrodèses interphalangiennes, pour bloquer l'articulation entre deux phalanges de la main ou du pied, en particulier dans le cadre du traitement des pathologies d'orteils en griffes.

Cet implant 1 est constitué d'un corps allongé d'axe longitudinal L, réalisé monobloc en tout matériau adapté, notamment en matériau métallique, en particulier en titane (par exemple du TI-6AL-6V).

Cet implant monobloc 1 comprend :
- une première partie d'ancrage d'extrémité 2, de forme générale plane, qui s'étend dans un plan P1, apte à venir s'ancrer dans le canal médullaire de l'une des phalanges concernées, en particulier, comme on le verra par la suite, apte à venir s'ancrer dans le canal médullaire d'une phalange proximale,
- une seconde partie d'ancrage d'extrémité 3, de forme générale plane, s'étendant dans un plan P2, apte à venir s'ancrer dans le canal médullaire de l'autre phalange concernée, en particulier, comme décrit plus loin, apte à venir s'ancrer dans le canal médullaire d'une phalange distale, et
- une partie intermédiaire 4, reliant de manière rigide lesdites première et seconde parties d'ancrage d'extrémité 2, 3.

Les première et seconde parties d'ancrage d'extrémité 2, 3 s'étendent dans des plans P1 et P2 perpendiculaires entre eux, pour résister aux forces rotationnelles. Une fois l'implant en place, on limite ainsi les possibilités de rotation des deux phalanges, ce qui permet en particulier d'améliorer la tenue dans le temps de l'implant 1 une fois posé.

D'autre part, la partie intermédiaire 4 est structurée pour constituer une butée permettant de faciliter et de sécuriser la pose de l'implant 1.

De plus, la structure des première et seconde parties d'ancrage 2, 3 permet un accrochage optimisé dans l'os.

Comme on peut le voir sur les figures 1 à 3, la première partie d'ancrage d'extrémité 2 se présente sous la forme générale d'un U comportant une embase 5 raccordée à la partie intermédiaire 4, et deux bras allongés 6a, 6b, parallèles ou sensiblement parallèles entre eux.

Ces deux bras 6a, 6b ont chacun une section carrée ou rectangulaire. Ils sont chacun délimités par deux faces planes 6a1, 6a2 ; 6b1, 6b2 qui sont parallèles entre elles et parallèles au plan P1, par une bordure extérieure 6a3, 6b3 orientées à l'opposé l'une de l'autre, et par une bordure intérieure 6a4, 6b4 disposées en regard l'une de l'autre.

La section des bras 6a, 6b est à peu près constante sur leur longueur.

Les bordures extérieures 6a3 et 6b3 des bras 6a et 6b définissent les bordures latérales de la première partie d'ancrage d'extrémité 2.

Les deux bras 6a et 6b de cette première partie d'ancrage 2 disposent chacun d'une possibilité de mobilité élastique par rapport à leur embase 5, cela dans le plan P1, et ils ont donc la possibilité de se rapprocher et de s'écarter légèrement l'un de l'autre sous l'effet de forces latérales.

Cette mobilité élastique est obtenue par la structure fine des bras 6a et 6b raccordés à l'embase 5, par leur design, et par leur matériau constitutif.

Sur les bordures extérieures 6a3 et 6b3 des deux bras 6a et 6b, on remarque la présence :
- d'au moins une réservation 7a, 7b en forme de rainure localisée au niveau de la zone de raccordement entre chaque bras 6a, 6b et l'embase 5, pour optimiser l'ancrage au sein de l'os (en l'occurrence chaque bras 6a, 6b comporte ici deux réservations 7) ; et
- d'une pointe monobloc 8a, 8b localisée au niveau de l'extrémité libre desdits bras 6a, 6b ; les deux pointes 8a et 8b s'étendent dans le plan P1 et dans des directions opposées l'une de l'autre.

Sur les figures 1 et 3 on remarque encore la forme générale cintrée des deux bras 6a et 6b, avec leur partie concave correspondant à leur bordure extérieure 6a3, 6b3.

De ce fait, la première partie d'ancrage d'extrémité 2 comporte, dans le plan P1 et partant de l'embase 5 :
- une partie initiale relativement large/évasée (correspondant à la partie sur laquelle sont ménagées les réservations 7a, 7b), au niveau de laquelle les deux bras 6a, 6b sont assez écartés l'un de l'autre,
- une partie centrale peu large au niveau de laquelle les deux bras 6a, 6b sont plutôt rapprochés l'un de l'autre, et
- une partie terminale relativement large/évasée, au niveau de laquelle les deux bras 6a, 6b sont assez écartés l'un de l'autre et au niveau de laquelle se situent les pointes en saillie 8a, 8b.

Les parties initiale et terminale précitées ont une largeur sensiblement identique, la partie centrale ayant une largeur plus petite.

La seconde partie d'ancrage d'extrémité 3 se présente sous la forme d'une platine 10 qui est raccordée à la partie intermédiaire 4 par une embase 11.
Cette platine 10 est de forme générale rectangulaire et elle est délimitée :
- par deux faces 10a, 10b parallèles entre elles et parallèles au plan P2,
- par deux bordures latérales parallèles 10c, 10d munies d'une pluralité de dents juxtaposées 12, et
- par une extrémité libre 13 en forme de Vé ou de pointe.

Une telle structure de cette partie d'ancrage d'extrémité assure une bonne résistance et une bonne tenue dans le canal médullaire de réception.

D'autre part, l'implant 1 comporte des moyens pour sa préhension par un matériel adapté permettant sa pose lors de l'intervention chirurgicale.

Ces moyens de préhension sont ici prévus au niveau de la seconde partie d'ancrage 3 et consistent :
- en un orifice traversant 14, de forme circulaire ou oblongue, ménagé dans la platine 10, à proximité de l'embase 11, et qui débouche dans les faces 10a, 10b de ladite platine 10, et
- en deux appuis latéraux 15, ici en forme d'arc de cercle, ménagés sur les côtés de l'embase 11, dans le prolongement des bordures latérales 10c, 10d de la platine 10, entre ledit orifice traversant 14 et la partie intermédiaire 4.

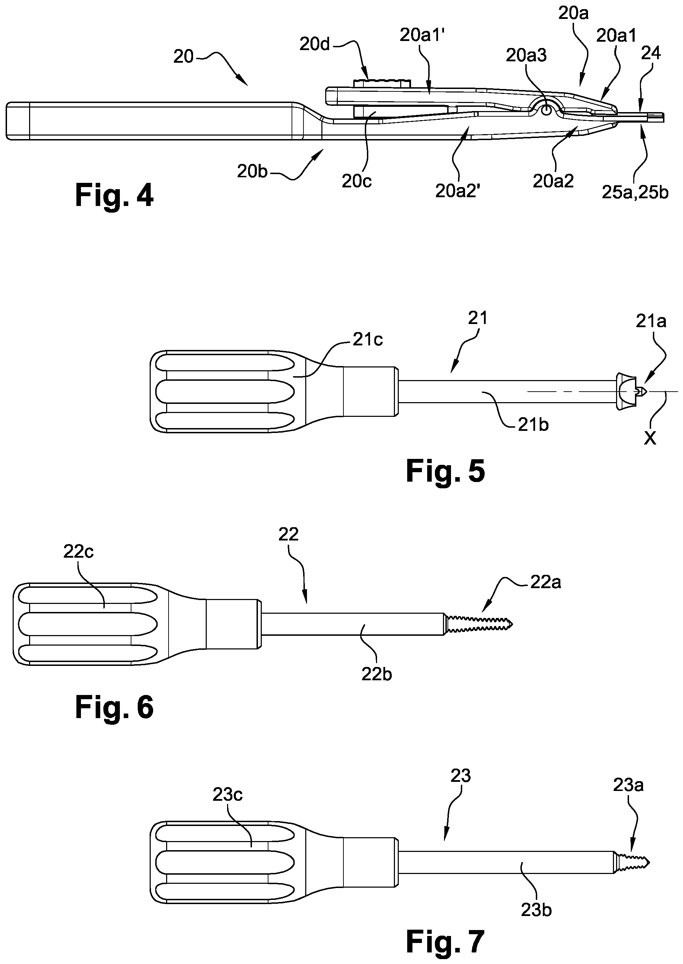

Le matériel de pose en question est détaillé plus loin dans la description, en relation avec les figures 4 à 11.

La partie intermédiaire 4 qui relie les deux parties d'ancrage d'extrémité 2 et 3 comporte une structure monobloc en forme de plateau 16 qui s'étend dans un plan P3 perpendiculaire à l'axe longitudinal L, et perpendiculaire aux deux plans P1 et P2 précités.

Le plateau 16 a une forme générale rectangulaire aux angles duquel sont ménagées quatre réservations d'allègement 17. Il comporte une face 16a orientée vers la première partie d'ancrage d'extrémité 2 et une face 16b orientée vers la seconde partie d'ancrage d'extrémité 3.

Ce plateau 16 de la partie intermédiaire 4 est centré sur l'axe longitudinal **L** et cet axe longitudinal L correspond à l'axe médian des deux structures d'ancrage d'extrémité 2 et 3.

La pose de l'implant intramédullaire 1 est avantageusement réalisée au moyen d'un matériel de pose comportant :
- un dispositif de préhension en forme de pince de préhension 20 dont une forme de réalisation possible est représentée sur la figure 4,
- une fraise de résection 21 dont une forme de réalisation possible est illustrée sur la figure 5, pour la résection des extrémités des deux phalanges concernées, et
- deux râpes 22, 23 dont des exemples de réalisation possibles sont illustrés sur les figures 6 et 7, pour la préparation des cavités de réception de l'implant 1, respectivement dans les phalanges proximale et distale.

La pince de préhension 20 (figure 4) comprend une tête 20a, à l'avant, prolongée par un manche 20b à l'arrière. La tête 20a est munie de deux mâchoires 20a1 et 20a2 articulées entre elles au niveau d'un point pivot 20a3.

Chaque mâchoire 20a1 et 20a2 se prolonge vers l'arrière, au-delà du point pivot 20a3, respectivement par un bras 20a1' et 20a2' constituant le manche 20b.

La première mâchoire 20a1 comporte une tige 24 en saillie, apte à venir se loger dans l'orifice traversant 14 de la platine 10 de la seconde partie d'accrochage d'extrémité 3. La section de cette tige 24 peut être circulaire ; et cette section de la tige 24 est inférieure à la section de l'orifice traversant 14.

La seconde mâchoire 20a2 comporte de son côté deux bras parallèles ou sensiblement parallèles 25a, 25b, en saillie, aptes chacun à venir coopérer avec l'un des appuis latéraux 15 de la seconde partie d'ancrage d'extrémité 3 de l'implant 1. Les deux bras 25a et 25b s'étendent dans un plan qui est parallèle au point pivot 20a3.

La section des deux bras 25a et 25b peut être circulaire ou légèrement allongée. Leurs surfaces orientées en regard sont complémentaires de celle des appuis latéraux 15 (c'est-à-dire ici à section en arc de cercle).

Les deux bras 25a, 25b et la tige en saillie 24 s'étendent parallèlement ou sensiblement parallèlement entre eux ; les mâchoires 20a, 20b qui les supportent permettent d'écarter ou de rapprocher légèrement la tige 24 par rapport aux deux bras 25a, 25b, pour permettre la préhension et la libération de l'implant 1 de la manière décrite plus loin.

Le manche 20b comporte une cale mobile 20c, disposée entre les deux branches 20a1' et 20a2', actionnable en coulissement longitudinal par un bouton de manoeuvre 20d, pour rapprocher ou écarter les deux mâchoires 20a1 et 20a2. Cette cale 20c comporte des moyens qui permettent de verrouiller au moins la position active de préhension de l'implant 1.

De plus, une butée mécanique peut être prévue pour ne pas permettre un enfoncement trop important de l'implant sur la tige 24. Cette butée peut consister en un épaulement ménagé sur la tige 24 pour assurer un maintien optimal de l'implant.

La fraise de résection 21 (figure 5) comporte une partie active de résection 21a montée à l'extrémité d'une tige 21b prolongée par un manche de manoeuvre 21c.

Ces trois parties 21a, 21b et 21c sont coaxiales le long d'un axe longitudinal X pour permettre les opérations de résection par rotation du manche 21c autour de cet axe X.

La râpe 22 illustrée sur la figure 6 (dite râpe « proximale ») comporte une partie active 22a agencée à l'extrémité d'une tige 22b prolongée par un manche 22c.

La partie active 22a de la râpe 22 est de forme générale plate triangulaire, munie de bordures latérales dentées. Sa forme générale et ses dimensions sont adaptées à celles de la cavité que l'on souhaite réaliser dans la phalange proximale pour l'accueil de la première partie d'ancrage d'extrémité 2 de l'implant 1.

La partie active 22a est placée dans l'axe de la tige 22b. L'extrémité de cette tige 22b qui est raccordée à la partie active 22a constitue une butée apte à limiter l'enfoncement axial de ladite partie active 22a.

La râpe 23 illustrée sur la figure 7 (dite râpe « distale ») comporte une partie active 23a agencée à l'extrémité d'une tige 23b prolongée par un manche 23c.

La partie active 23a de la râpe 23 est de forme générale plate triangulaire munie de bordures latérales dentées. Sa forme générale et ses dimensions sont adaptées à celles de la cavité que l'on souhaite réaliser dans la phalange distale pour l'accueil de la seconde partie d'ancrage d'extrémité 3 de l'implant 1.

La partie active 23a est placée dans l'axe de la tige 23b. L'extrémité de cette tige 23b qui est raccordée à la partie active 23a constitue une butée apte à limiter l'enfoncement axial de ladite partie active 23a.

La technique de pose de l'implant intramédullaire 1 consiste dans un premier temps à préparer les extrémités en regard des deux phalanges concernées.

Après réalisation de l'incision dorsale standard au niveau de l'articulation, et le positionnement orthogonal des deux phalanges, l'opérateur résèque l'extrémité proximale de la phalange distale et l'extrémité distale de la phalange proximale, puis il avive doucement les deux surfaces au moyen de la fraise 21.

Ensuite, il insère jusqu'en butée la râpe « proximale » 22 par l'extrémité distale réséquée de la phalange proximale, en prenant soin de respecter l'orientation future de l'implant 1 (c'est-à-dire ici en orientant le plan de la râpe 22 dans le plan sagittal de la phalange), et il insère jusqu'en butée la râpe « distale » 23 par l'extrémité proximale réséquée de la phalange distale, en prenant soin de respecter l'orientation future de l'implant 1 (c'est-à-dire ici en orientant le plan de la râpe 23 dans le plan frontal de la phalange).

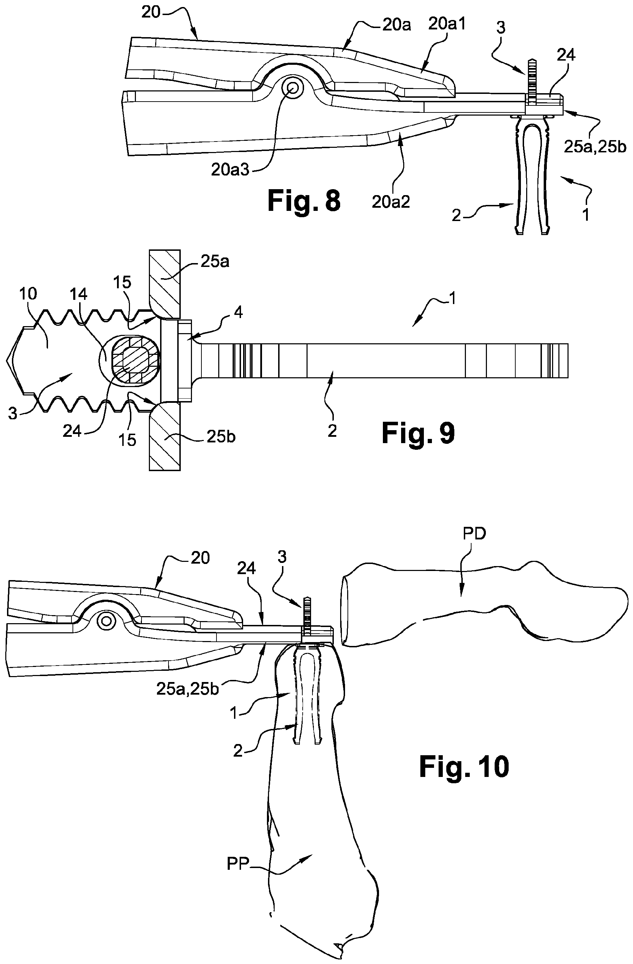

L'opérateur monte ensuite l'implant 1 dans la pince de préhension 20 de la manière illustrée sur les figures 8 et 9, puis il positionne l'implant entre et au sein des phalanges proximale PP et distale PD de la manière illustrée sur les figures 10 à 12.

L'opération de montage de l'implant 1 dans la pince de préhension 20 est réalisée en présentant la tige 24 et les bras 25a, 25b perpendiculairement à l'axe longitudinal L de l'implant 1, et en insérant la partie distale de la tige 24 dans l'orifice traversant 14 de la platine 10.

Au cours de cette insertion, les bras 25a et 25b de la pince de préhension 20 viennent se positionner chacun en regard de l'un des appuis latéraux 15, sans appui ou tout au moins sans appui serré contre ces derniers.

Le blocage de l'implant 1 dans la pince de préhension 20 est obtenu en activant la cale de serrage 20c, par coulissement vers l'avant du bouton de manoeuvre 20d, assurant la fermeture des mâchoires 20a, 20b. Cette manoeuvre provoque le rapprochement de la tige 24 et des deux bras 25a, 25b, avec appui de la tige 24 contre la surface interne de l'orifice traversant 24 (côté partie intermédiaire 4), et appui des bras 25a, 25b contre les appuis latéraux 15 de l'implant 1 (figures 8 et 9).

La première partie d'ancrage d'extrémité 2 et la partie intermédiaire 4 de l'implant 1 s'étendent d'un côté des tige 24 et bras 25a, 25b, et une majeure partie de la seconde partie d'ancrage d'extrémité 3 s'étend de l'autre côté desdites tige 24 et bras 25a, 25b.

La tige 24 et les bras 25a, 25b occupent un encombrement relativement réduit sur l'axe longitudinal L de l'implant 1.

Une fois l'implant 1 monté sur la pince de préhension 20, l'opérateur insère la première partie d'ancrage d'extrémité 2 au sein de la cavité ménagée dans la phalange proximale PP, comme illustré sur la figure 10, jusqu'à ce que la face 16a du plateau 16 de la partie intermédiaire 4 vienne en butée contre l'os.

La cavité ménagée dans l'os est adaptée pour que les deux bras 6a et 6b de la première partie d'ancrage d'extrémité 2 se resserrent légèrement et exercent par élasticité une pression contre la corticale osseuse, notamment au niveau des deux pointes 8a, 8b, augmentant ainsi la stabilité de l'implant.

Le plan P1 de la première partie d'ancrage d'extrémité 2 s'étend alors dans le plan sagittal de la phalange proximale PP.

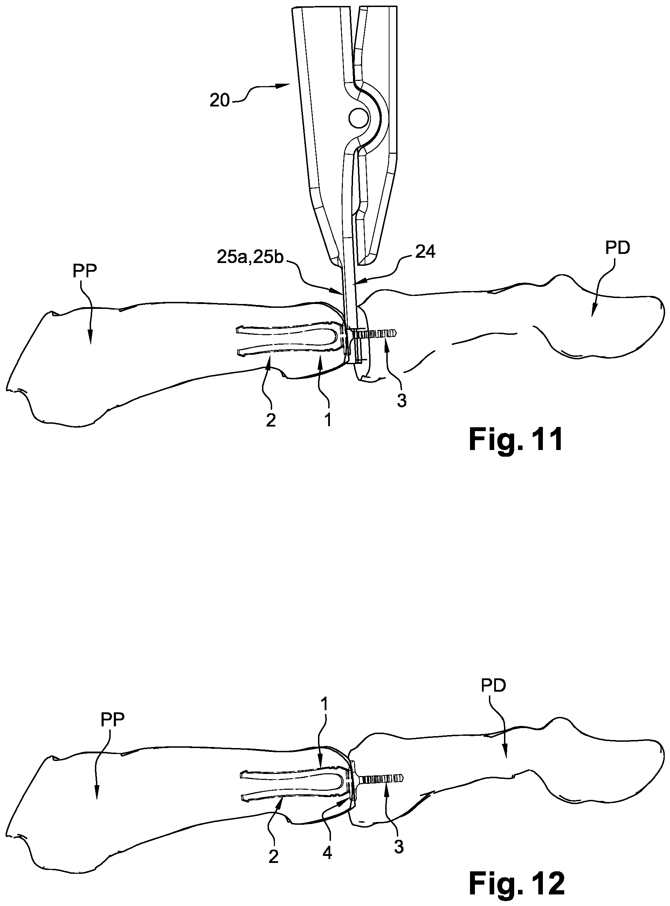

Ensuite l'opérateur réduit manuellement la phalange distale sur la partie « distale » de l'implant 1 (à savoir sur la seconde partie d'ancrage d'extrémité 3), cela tout en maintenant positionnée la pince de préhension 20 (figure 11) de façon à conserver la stabilité et le contrôle du montage pendant la réduction.

Ce maintien en position de la pince de préhension 20 est possible du fait du faible encombrement de la tige 24 et des bras 25a, 25b au sein de la zone interphalangienne.

La pince de préhension 20 est retirée par desserrage de ses mâchoires 20a, 20b et extraction perpendiculairement à l'axe longitudinal L de l'implant. La réduction est finalisée par l'opérateur en s'assurant du contact entre elles des phalanges proximale PP et distale PD (et du contact des deux phalanges PP et PD avec les faces en regard 16a, 16b de la partie intermédiaire 4 de l'implant 1).

Le positionnement du plan P2 de la seconde partie d'ancrage d'extrémité 3 dans le plan frontal de la phalange distale PD et parallèlement à l'articulation phalangienne permet d'assurer une résistance optimale de l'implant aux efforts de flexion engendrés sur la phalange distale lors du cycle de marche.

L'implant 1 conforme à l'invention présente de nombreux avantages, en particulier :
- les propriétés élastiques des bras 6a et 6b de l'implant 1 permettent un accrochage optimisé dans l'os, les rainures 7a, 7b de la première partie d'ancrage d'extrémité 2 et les dents 12 de la seconde partie d'ancrage d'extrémité 3 assurant une bonne résistance à l'arrachement.
- un tel implant 1 monobloc et avec des première et seconde parties d'ancrage d'extrémité 2, 3 orientées perpendiculairement l'une par rapport à l'autre, permet de limiter le risque de luxation et de rotation des deux phalanges.
- la seconde partie d'ancrage d'extrémité 3 en forme de platine 10 permet d'améliorer la résistance structurelle de l'implant.
- le positionnement d'un tel implant interphalangien est rapide et sécurisé grâce à sa butée centrale 4 et à la pince de préhension 20.

On notera que dans des variantes de réalisation, le plan P2 de la seconde partie d'ancrage d'extrémité 3 peut être positionné de manière légèrement inclinée par rapport à l'axe longitudinal L, pour fixer une angulation entre les deux phalanges concernées PP et PD.

## Revendications

1. Implant intramédullaire (1) pour le blocage de l'articulation entre deux phalanges (PP, PD) de la main ou du pied, lequel implant (1) est constitué d'un corps allongé monobloc d'axe longitudinal (L) comprenant :
- une première partie d'ancrage d'extrémité (2) de forme générale plane, s'étendant dans un plan (P1), apte à venir s'ancrer dans le canal médullaire de l'une desdites phalanges (PP), et
- une seconde partie d'ancrage d'extrémité (3), de forme générale plane, s'étendant dans un plan (P2), apte à venir s'ancrer dans le canal médullaire de l'autre desdites phalanges (PD), et
- une partie intermédiaire (4) reliant de manière rigide lesdites première et seconde parties d'ancrage d'extrémité (2, 3),
lesquelles première et seconde parties d'ancrage d'extrémité (2, 3) s'étendent dans des plans (P1) et (P2) qui sont perpendiculaires ou sensiblement perpendiculaires entre eux,
et laquelle première partie d'ancrage d'extrémité (2) est en forme générale de U comportant une embase (5) raccordée à ladite partie intermédiaire (4), et deux bras (6a, 6b) s'étendant parallèlement ou sensiblement parallèlement entre eux à partir de ladite embase (5),
**caractérisé en ce que** ladite seconde partie d'ancrage d'extrémité (3) est en forme de platine (10) raccordée à ladite partie intermédiaire (4) par une embase (11), laquelle platine (10) est délimitée par deux faces (10a, 10b) parallèles entre elles et parallèles audit plan (P2), par une extrémité libre (13) et par deux bordures latérales (10c, 10d), lesquelles bordures latérales (10c, 10d) comportent une pluralité de dents (12) juxtaposées.

2. Implant intramédullaire (1) selon la revendication 1, **caractérisé en ce qu'**il comporte au moins une réservation (7a, 7b) constituant un amincissement de matière au niveau de la zone de raccordement entre ladite embase (5) et chacun desdits bras (6a, 6b), pour optimiser l'ancrage au sein de l'os.

3. Implant intramédullaire (1) selon l'une quelconque des revendications 1 ou 2, **caractérisé en ce que** lesdits bras (6a, 6b) sont chacun délimités par deux faces planes (6a1, 6a2; 6b1, 6b2) parallèles entre elles et parallèles audit plan (P1), par une bordure extérieure (6a3, 6b3) et par une bordure intérieure (6a4, 6b4), lesdites bordures intérieures (6a4, 6b4) des deux bras (6a, 6b) étant en regard l'une de l'autre, et lesdites bordures extérieures (6a3, 6b3) des deux bras (6a, 6b) définissant les bordures latérales de ladite première partie d'ancrage d'extrémité (2).

4. Implant intramédullaire (1) selon l'une quelconque des revendications 1 à 3, **caractérisé en ce que** lesdits bras (6a, 6b) disposent chacun d'une possibilité de mobilité axiale par rapport à ladite embase (5), cela dans ledit plan (P1), et **en ce que** lesdits bras (6a, 6b) comportent chacun une bordure extérieure (6a3, 6b3) et une bordure intérieure (6a4, 6b4), lesdites bordures intérieures (6a4, 6b4) des deux bras (6a, 6b) étant en regard l'une de l'autre et lesdites bordures extérieures (6a3, 6b3) des deux bras (6a, 6b) définissant les bordures latérales de ladite première partie d'ancrage d'extrémité (2),
et **en ce que** lesdits bras (6a, 6b) ont chacun une forme générale cintrée dont la partie concave correspond à ladite bordure extérieure (6a3, 6b3).

5. Implant intramédullaire (1) selon l'une quelconque des revendications 3 ou 4, **caractérisé en ce que** les extrémités libres des deux bras (6a, 6b) comportent chacun une pointe monobloc (8a, 8b) s'étendant en saillie au niveau de ladite bordure extérieure (6a3, 6b3), lesdites pointes (8a, 8b) des deux bras (6a, 6b) s'étendant dans ledit plan (P1) et dans des directions opposées.

6. Implant intramédullaire (1) selon la revendication 1, **caractérisé en ce que** ladite platine (10) est de forme générale rectangulaire.

7. Implant intramédullaire (1) selon l'une quelconque des revendications 1 ou 6, **caractérisé en ce que** l'extrémité libre (13) de ladite platine (10) est en forme de pointe.

8. Implant intramédullaire (1) selon l'une quelconque des revendications 1 ou 6 ou 7, **caractérisé en ce qu'**il comporte des moyens (14, 15) pour sa préhension par un matériel adapté, lesquels moyens consistent en un orifice traversant (14) ménagé dans ladite platine (10) à proximité de ladite embase (11), et en deux appuis latéraux (15) ménagés sur les côtés de ladite embase (11), entre ledit orifice traversant (14) et ladite partie intermédiaire (4).

9. Implant intramédullaire (1) selon l'une quelconque des revendications 1 à 8, **caractérisé en ce que** ladite partie intermédiaire (4) comporte une structure monobloc (16) qui s'étend dans un plan (P3) perpendiculaire audit axe longitudinal (L).

10. Implant intramédullaire (1) selon la revendication 9, **caractérisé en ce que** ladite structure monobloc est en forme générale de plateau (16) centré sur ledit axe longitudinal (L).

11. Implant intramédullaire (1) selon la revendication 10, **caractérisé en ce que** ladite structure monobloc est en forme générale de plateau rectangulaire (16) comportant quatre réservations (17) au niveau de ses angles.

12. Implant intramédullaire (1) selon l'une quelconque des revendications 1 à 11, **caractérisé en ce que** ladite première partie d'ancrage d'extrémité (2) et adaptée pour constituer la partie proximale d'ancrage destinée à venir s'ancrer dans le canal médullaire de la phalange proximale (PP) de l'articulation, et **en ce que** ladite seconde partie d'ancrage d'extrémité (3) est adaptée pour constituer la partie distale d'ancrage, destinée à venir s'ancrer dans le canal médullaire de la phalange distale (PD).

13. Dispositif d'ostéosynthèse ou d'arthrodèse comportant :
a/ un implant intramédullaire (1), selon la revendication 8, et
b/ un dispositif de pose pour ledit implant intramédullaire (1), qui se présente sous la forme d'une pince de préhension (20) munie de deux mâchoires articulées (20a, 20b),
- une première desdites mâchoires (20a) comportant une tige (24) apte à venir se loger dans l'orifice traversant (14) de ladite platine (10) de ladite seconde partie d'ancrage d'extrémité (3), et
- une seconde desdites mâchoires (20b) comportant deux bras (25a, 25b) parallèles ou sensiblement parallèles, aptes chacun à venir coopérer avec l'un desdits appui latéraux (15) de ladite seconde partie d'ancrage d'extrémité (3).

14. Dispositif d'ostéosynthèse ou d'arthrodèse selon la revendication 13, **caractérisé en ce qu'**il comporte encore :
- une fraise de résection (21) comportant une partie active (21a) montée à l'extrémité d'une tige (21b) munie d'un manche (21c),
- une râpe proximale (22) comportant une partie active (22a) adaptée pour la préparation d'un logement intramédullaire dans la phase proximale (PP), en vue de la réception de la partie proximale d'ancrage (3) de l'implant intramédullaire (1), et
- une râpe distale (23) comportant une partie active (23a) adaptée pour la préparation d'un logement intramédullaire dans la phalange distale (PD), en vue de la réception de la partie distale d'ancrage (3) de l'implant intramédullaire (1).

## Patentansprüche

1. Intramedulläres Implantat (1) zur Ruhigstellung des Gelenks zwischen zwei Phalangen (PP, PD) der Hand oder des Fußes, wobei das Implantat (1) aus einem einstückigen länglichen Körper mit Längsachse (L) besteht und
- ein erstes Endverankerungsteil (2) von im Wesentlichen flacher Form, das sich in einer Ebene (P1) erstreckt und dazu ausgelegt ist, im Medullärkanal einer der Phalangen (PP) verankert zu werden, und
- ein zweites Endverankerungsteil (3) von im Wesentlichen flacher Form, das sich in einer Ebene (P2) erstreckt und dazu ausgelegt ist, im Medullärkanal der anderen der Phalangen (PD) verankert zu werden, und
- ein Zwischenteil (4), das das erste und das zweite Endverankerungsteil (2, 3) fest miteinander verbindet,
aufweist,
wobei sich das erste und das zweite Endverankerungsteil (2, 3) in Ebenen (P1) und (P2) erstrecken, die zueinander senkrecht oder im Wesentlichen zueinander senkrecht sind,
und wobei das erste Endverankerungsteil (2) im Wesentlichen U-förmig ist und eine mit dem Zwischenteil (4) verbundene Basis (5) und zwei sich von der Basis (5) aus zueinander parallel oder im Wesentlichen zueinander parallel erstreckende Arme (6a, 6b) aufweist,
**dadurch gekennzeichnet, daß** das zweite Endverankerungsteil (3) die Form einer Platine (10) aufweist, die mit dem Zwischenteil (4) über eine Basis (11) verbunden ist, wobei die Platine (10) von zwei zueinander parallelen und zur Ebene (P2) parallelen Seiten (10a, 10b), von einem freien Ende (13) und von zwei seitlichen Rändern (10c, 10d) begrenzt ist, wobei die seitlichen Ränder (10c, 10d) eine Anzahl nebeneinander angeordneter Zähne (12) aufweisen.

2. Intramedulläres Implantat (1) gemäß Anspruch 1, **dadurch gekennzeichnet, daß** es wenigstens eine im Bereich der Verbindung zwischen der Basis (5) und jedem der Arme (6a, 6b) eine Materialverringerung bildende Ausnehmung (7a, 7b) aufweist, um die Verankerung im Knochen zu optimieren.

3. Intramedulläres Implantat (1) gemäß einem der Ansprüche 1 oder 2, **dadurch gekennzeichnet, daß** die Arme (6a, 6b) jeweils von zwei zueinander parallelen und zur Ebene (P1) parallelen flachen Seiten (6a1, 6a2; 6b1, 6b2), von einem äußeren Rand (6a3, 6b3) und von einem inneren Rand (6a4, 6b4) begrenzt sind, wobei sich die inneren Ränder (6a4, 6b4) der beiden Arme (6a, 6b) gegenüberliegen und wobei die äußeren Ränder (6a3, 6b3) der beiden Arme (6a, 6b) die seitlichen Ränder des ersten Endverankerungsteils (2) definieren.

4. Intramedulläres Implantat (1) gemäß einem der Ansprüche 1 bis 3, **dadurch gekennzeichnet, daß** die Arme (6a, 6b) jeweils eine Möglichkeit einer axialen Beweglichkeit gegenüber der Basis (5) aufweisen, und zwar in der Ebene (P1), daß die Arme (6a, 6b) jeweils einen äußeren Rand (6a3, 6b3) und einen inneren Rand (6a4, 6b4) aufweisen, wobei sich die inneren Ränder (6a4, 6b4) der beiden Arme (6a, 6b) gegenüberliegen und wobei die äußeren Ränder (6a3, 6b3) der beiden Arme (6a, 6b) die seitlichen Ränder des ersten Endverankerungsteils (2) definieren,
und daß die Arme (6a, 6b) jeweils eine allgemeine gebogene Form aufweisen, deren konkaver Teil dem äußeren Rand (6a3, 6b3) entspricht.

5. Intramedulläres Implantat (1) gemäß einem der Ansprüche 3 oder 4, **dadurch gekennzeichnet, daß** die freien Enden der beiden Anne (6a, 6b) jeweils eine einstückig ausgebildete Spitze (8a, 8b) aufweisen, die sich im Bereich des äußeren Rands (6a3, 6b3) hervorstehend erstreckt, wobei sich die Spitzen (8a, 8b) der beiden Arme (6a, 6b) in der Ebene (P1) und in entgegengesetzten Richtungen erstrecken.

6. Intramedulläres Implantat (1) gemäß Anspruch 1, **dadurch gekennzeichnet, daß** die Platine (10) von allgemeiner rechteckiger Form ist.

7. Intramedulläres Implantat (1) gemäß einem der Ansprüche 1 oder 6, **dadurch gekennzeichnet, daß** das freie Ende (13) der Platine (10) die Form einer Spitze aufweist.

8. Intramedulläres Implantat (1) gemäß einem der Ansprüche 1 oder 6 oder 7, **dadurch gekennzeichnet, daß** es Mittel (14, 15) für dessen Erfassen mittels eines geeigneten Gegenstands aufweist, wobei die Mittel aus einem in der Nähe der Basis (11) in die Platine (10) eingebrachten Durchgangsloch (14) und aus zwei auf den Seiten der Basis (11) zwischen dem Durchgangsloch (14) und dem Zwischenteil (4) eingerichteten seitlichen Abstützungen (15) bestehen.

9. Intramedulläres Implantat (1) gemäß einem der Ansprüche 1 bis 8, **dadurch gekennzeichnet, daß** das Zwischenteil (4) eine einstückige Struktur (16) aufweist, die sich in einer zur Längsachse (L) senkrechten Ebene (P3) erstreckt.

10. Intramedulläres Implantat (1) gemäß Anspruch 9, **dadurch gekennzeichnet, daß** die einstückige Struktur die allgemeine Form einer Platte (16) aufweist, die auf die Längsachse (L) zentriert ist.

11. Intramedulläres Implantat (1) gemäß Anspruch 10, **dadurch gekennzeichnet, daß** die einstückige Struktur die allgemeine Form einer rechteckigen Platte (16) aufweist, die im Bereich ihrer Ecken vier Aüsnchmungen (17) aufweist.

12. Intramedulläres Implantat (1) gemäß einem der Ansprüche 1 bis 11, **dadurch gekennzeichnet, daß** das erste Endverankerungsteil (2) dazu ausgelegt ist, das proximale Verankerungsteil zu bilden, das dazu bestimmt ist, im Medullärkanal der proximalen Phalange (PP) des Gelenks verankert zu werden, und daß das zweite Endverankerungsteil (3) dazu ausgelegt ist, das distale Verankerungsteil zu bilden, das dazu bestimmt ist, im Medullärkanal der distalen Phalange (PD) verankert zu werden.

13. Osteosynthese- oder Arthrodesevorrichtung mit
a) einem intramedullären Implantat (1) gemäß Anspruch 8 und
b) einer Einsetzvorrichtung für das intramedulläre Implantat (1), die in Form einer mit zwei angelenkten Backen (20a, 20b) versehenen Greifzange (20) vorliegt,
- wobei eine erste (20a) der Backen einen Stab (24) aufweist, der dazu ausgelegt ist, in das Durchgangsloch (14) der Platine (10) des zweiten Endverankerungsteils (3) einzugreifen, und
- eine zweite (20b) der Backen zwei parallele oder im Wesentlichen parallele Arme (25a, 25b) aufweist, von denen jeder dazu ausgelegt ist, mit einer der seitlichen Abstützungen (15) des zweiten Endverankerungsteils (3) zusammenzuwirken.

14. Osteosynthese- oder Arthrodesevorrichtung gemäß Anspruch 13, **dadurch gekennzeichnet, daß** sie außerdem
- einen Resektionsfräser (21), der einen am Ende einer mit einem Griff (21c) versehenen Stange (21b) angebrachten aktiven Teil (21a) aufweist,
- eine proximale Raspel (22), die einen aktiven Teil (22a) aufweist, der im Hinblick auf das Aufnehmen des proximalen Endverankerungsteils (2) des intramedullären Implantats (1) für das Vorbereiten einer intramedullären Aufnahme in der proximalen Phalange (PP) ausgelegt ist, und
- eine distale Raspel (23), die einen aktiven Teil (23a) aufweist, der im Hinblick auf das Aufnehmen des distalen Endverankerungsteils (3) des intramedullären Implantats (1) für das Vorbereiten einer intramedullären Aufnahme in der distalen Phalange (PD) ausgelegt ist,
aufweist.

## Claims

1. An intramedullary implant (1) for locking the articulation between two phalanges (PP, PD) of the hand or the foot, wherein said implant (1) is consisted of a single-piece elongated body of longitudinal axis (L) comprising:
- a first end anchoring portion (2), generally planar in shape, extending in a plane (P1), adapted to be anchored into the medullary canal of one of said phalanges (PP), and
- a second end anchoring portion (3), generally planar in shape, extending in a plane (P2), adapted to be anchored into the medullary canal of the other of said phalanges (PD), and
- an intermediate portion (4) rigidly connecting said first and second end anchoring portions (2, 3),
wherein said first and second end anchoring portions (2, 3) extend in planes (P1) and (P2) that are perpendicular or substantially perpendicular to each other,
and wherein said first end anchoring portion (2) is generally U-shaped, including a base (5) connected to said intermediate portion (4), and two arms (6a, 6b) extending parallel or substantially parallel to each other from said base (5),
**characterized in that** said second end anchoring portion (3) has the shape of a plate (10) connected to said intermediate portion (4) by a base (11), wherein said plate (10) is delimited by two faces (10a, 10b) parallel to each other and parallel to said plane (P2), by a free end (13) and by two lateral edges (10c, 10d), wherein said lateral edges (10c, 10d) include a plurality of juxtaposed teeth (12)

2. The intramedullary implant (1) according to claim 1, **characterized in that** it includes at least one recess (7a, 7b) forming a material thinning in the area of connection between said base (5) and each of said arms (6a, 6b), to optimize the anchoring within the bone.

3. The intramedullary implant (1) according to any one of claims 1 or 2, **characterized in that** said arms (6a, 6b) are each delimited by two planar faces (6a1, 6a2; 6b1, 6b2) parallel to each other and parallel to said plane (P1), by an outer edge (6a3, 6b3) and by an inner edge (6a4, 6b4), said inner edges (6a4, 6b4) of the two arms (6a, 6b) being opposite to each other, and said outer edges (6a3, 6b3) of the two arms (6a, 6b) defining the lateral edges of said first end anchoring portion (2).

4. The intramedullary implant (1) according to any one of claims 1 to 3, **characterized in that** said arms (6a, 6b) each have a possibility of axial mobility with respect to said base (5), in said plane (P1), and **in that** said arms (6a, 6b) each have an outer edge (6a3, 6b3) and an inner edge (6a4, 6b4), said inner edges (6a4, 6b4) of the two arms (6a, 6b) being opposite to each other and said outer edges (6a3, 6b3) of the two arms (6a, 6b) defining the lateral edges of said first end anchoring portion (2),
and **in that** said arms (6a, 6b) each have a generally curved shape whose concave portion corresponds to said outer edge (6a3, 6b3).

5. The intramedullary implant (1) according to any one of claims 3 or 4, **characterized in that** the free ends of the two arms (6a, 6b) each include an integral tip (8a, 8b) extending in protrusion at said outer edge (6a3, 6b3), said tips (8a, 8b) of the two arms (6a, 6b) extending in said plane (P1) and in opposite directions.

6. The intramedullary implant (1) according to claim 1, **characterized in that** said plate (10) is generally rectangular in shape.

7. The intramedullary implant (1) according to any one of claims 1 or 6, **characterized in that** the free end (13) of said plate (10) is pointed in shape.

8. The intramedullary implant (1) according to any one of claims 1 or 6 or 7, **characterized in that** it includes means (14, 15) for its gripping by a suitable material, said means consisted of a through-orifice (14) formed into said plate (10) near said base (11), and of two lateral supports (15) formed on the sides of said base (11), between said through-orifice (14) and said intermediate portion (4).

9. The intramedullary implant (1) according to any one of claims 1 to 8, **characterized in that** said intermediate portion (4) includes a single-piece structure (16) that extends in a plane (P3) perpendicular to said longitudinal axis (L).

10. The intramedullary implant (1) according to claim 9, **characterized in that** said single-piece structure has the general shape of a plate (16) centred on said longitudinal axis (L).

11. The intramedullary implant (1) according to claim 10, **characterized in that** said single-piece structure has the general shape of a rectangular plate (16) having four recesses (17) at its angles.

12. The intramedullary implant (1) according to any one of claims 1 to 11, **characterized in that** said first end anchoring portion (2) is adapted to form the proximal anchoring portion intended to be anchored into the medullary canal of the proximal phalange (PP) of the articulation, and **in that** said second end anchoring portion (3) is adapted to form the distal anchoring portion, intended to be anchored into the medullary canal of the distal phalange (PD).

13. An osteosynthesis or arthrodesis device including:
a) an intramedullary implant (1) according to claim 8, and
b) a device for implanting said intramedullary implant (1), which is in the form of a gripping clamp (20) provided with two articulated jaws (20a, 20b),
- a first one of said jaws (20a) including a rod (24) adapted to be received into the through-orifice (14) of said plate (10) of said second end anchoring portion (3), and
- a second one of said jaws (20b) including two arms (25a, 25b) parallel or substantially parallel to each other, each adapted to cooperate with one of said lateral supports (15) of said second end anchoring portion (3).

14. The osteosynthesis or arthrodesis device according to claim 13, **characterized in that** it also includes:
- a resection burr (21) including an active portion (21a) mounted at the end of a rod (21b) provided with a handle (21c),
- a proximal grater (22) including an active portion (22a) suitable for preparing an intramedullary housing in the proximal phalange (PP), for receiving the proximal anchoring portion (3) of the intramedullary implant (1), and
- a distal grater (23) including an active portion (23a) suitable for preparing an intramedullary housing in the distal phalange (PD), for receiving the distal anchoring portion (3) of the intramedullary implant (1).
